(19) Europäisches Patentamt / European Patent Office / Office européen des brevets

(11) **EP 3 455 182 B1**

(12) **EUROPEAN PATENT SPECIFICATION**

(45) Date of publication and mention
of the grant of the patent:
**31.03.2021 Bulletin 2021/13**

(21) Application number: **17722054.8**

(22) Date of filing: **11.05.2017**

(51) Int Cl.:
*C03C 25/10* *(2018.01)*      *C03C 25/26* *(2018.01)*
*C09J 189/00* *(2006.01)*    *D04H 1/4209* *(2012.01)*
*D04H 1/64* *(2012.01)*      *D04H 3/002* *(2012.01)*
*D04H 3/004* *(2012.01)*    *D04H 1/4218* *(2012.01)*

(86) International application number:
**PCT/EP2017/061415**

(87) International publication number:
**WO 2017/194721 (16.11.2017 Gazette 2017/46)**

(54) **MINERAL WOOL PRODUCTS**

MINERALWOLLERZEUGNISSE

PRODUIT DE LAINE MINÉRALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2016 EP 16169635
13.05.2016 EP 16169641
13.05.2016 EP 16169638**

(43) Date of publication of application:
**20.03.2019 Bulletin 2019/12**

(73) Proprietor: **Rockwool International A/S
2640 Hedehusene (DK)**

(72) Inventor: **HJELMGAARD, Thomas
3480 Fredensborg (DK)**

(74) Representative: **Letzelter, Felix Phillip
Meissner Bolte Patentanwälte
Rechtsanwälte Partnerschaft mbB
Widenmayerstraße 47
80538 München (DE)**

(56) References cited:
**WO-A1-2010/125163     WO-A1-2016/005481**

## Description

### Field of the Invention

The present invention relates to a mineral wool product with a binder and a method of producing a mineral wool product with the binder.

### Background of the Invention

Mineral fibre products generally comprise man-made vitreous fibres (MMVF) such as, e.g., glass fibres, ceramic fibres, basalt fibres, slag wool, mineral wool and stone wool (rock wool), which are bonded together by a cured thermoset polymeric binder material. For use as thermal or acoustical insulation products, bonded mineral fibre mats are generally produced by converting a melt made of suitable raw materials to fibres in conventional manner, for instance by a spinning cup process or by a cascade rotor process. The fibres are blown into a forming chamber and, while airborne and while still hot, are sprayed with a binder solution and randomly deposited as a mat or web onto a travelling conveyor. The fibre mat is then transferred to a curing oven where heated air is blown through the mat to cure the binder and rigidly bond the mineral fibres together.

In the past, the binder resins of choice have been phenol-formaldehyde resins which can be economically produced and can be extended with urea prior to use as a binder. However, the existing and proposed legislation directed to the lowering or elimination of formaldehyde emissions have led to the development of formaldehyde-free binders such as, for instance, the binder compositions based on polycarboxy polymers and polyols or polyamines, such as disclosed in EP-A-583086, EP-A-990727, EP-A-1741726, US-A-5,318,990 and US-A-2007/0173588.

Another group of non-phenol-formaldehyde binders are the addition/-elimination reaction products of aliphatic and/or aromatic anhydrides with alkanolamines, e.g., as disclosed in WO 99/36368, WO 01/05725, WO 01/96460, WO 02/06178, WO 2004/007615 and WO 2006/061249. These binder compositions are water soluble and exhibit excellent binding properties in terms of curing speed and curing density. WO 2008/023032 discloses urea-modified binders of that type which provide mineral wool products having reduced moisture take-up.

Since some of the starting materials used in the production of these binders are rather expensive chemicals, there is an ongoing need to provide formaldehyde-free binders which are economically produced.

A further effect in connection with previously known aqueous binder compositions from mineral fibres is that at least the majority of the starting materials used for the productions of these binders stem from fossil fuels. There is an ongoing trend of consumers to prefer products that are fully or at least partly produced from renewable materials and there is therefore a need to provide binders for mineral wool which are at least partly produced from renewable materials.

A further effect in connection with previously known aqueous binder compositions for mineral fibres is that they involve components which are corrosive and/or harmful. This requires protective measures for the machinery involved in the production of mineral wool products to prevent corrosion and also requires safety measures for the persons handling this machinery. This leads to increased costs and health issues and there is therefore a need to provide binder compositions for mineral fibres with a reduced content of corrosive and/or harmful materials.

A yet further effect in connection with previously known aqueous binder compositions from mineral fibres is that these binders are conventionally associated with extensive curing equipment for curing the binder. The curing equipment is conventionally an oven operating at temperatures far above 100 °C such as around 200 °C. The oven is several meters long to accommodate the web that is continuously fed into the oven and to ensure that the web is fully cured when leaving the oven. Such oven equipment is associated with extensive energy consumption.

The reference EP 2424886 B1 (Dynea OY) describes a composite material comprising a crosslinkable resin of a proteinous material. In a typical embodiment, the composite material is a cast mould comprising an inorganic filler, like e.g. sand, and/or wood, and a proteinous material as well as enzymes suitable for crosslinking the proteinous material. A mineral wool product is not described in EP 2424886 B1.

WO 2010/125163 A1 relates to a composite material comprising filler and a resin comprising proteinous material cross-linkable by enzymes, and enzymes suitable for said crosslinking. The composite material is useful for making foundry moulds or wood panels.

WO 2016/005481 A1 is directed to a method of manufacturing a product selected from a building product, a mineral wool insulation product, a wood product, an automotive product, a paper product and a factory product comprising the step of applying an aqueous binder composition to a collection of matter whereby the aqueous binder composition comprises whey protein, carbohydrate, fat, and ash.

### Summary of the Invention

Accordingly, it was an object of the present invention to provide a mineral wool product comprising mineral fibers bound by a cured binder, wherein the binder uses renewable materials as starting materials, and reduces or eliminates corrosive and/or harmful materials.

Further, it was an object of the present invention to provide a mineral wool product comprising mineral fibres bound by a cured binder, which does not require high temperatures for curing during the preparation of the product.

A further object of the present invention was to provide a method of making such a mineral wool product.

In accordance with a first aspect of the present invention, there is provided a mineral wool product comprising mineral fibres bound by a cured binder wherein the binder in its uncured state comprises:

- at least one protein,
- at least one enzyme,

wherein the at least one protein is selected from the group consisting of proteins from animal sources, including collagen, gelatine, hydrolysed gelatine, and protein from milk (casein, whey), eggs; proteins from vegetable sources, including proteins from legumes, cereals, whole grains, nuts, seeds and fruits, like protein from buckwheat, oats, rye, millet, maize (corn), rice, wheat, bulgar, sorghum, amaranth, quinoa, soybeans (soy protein), lentils, kidney beans, white beans, mung beans, chickpeas, cowpeas, lima beans, pigeon peas, lupines, wing beans, almonds, Brazil nuts, cashews, pecans, walnuts, cotton seeds, pumpkin seeds, hemp seeds, sesame seeds, and sunflower seeds; polyphenolic proteins such as mussel foot protein,

wherein the at least one enzyme is selected from the group consisting of transglutaminase (EC 2.3.2.13), protein disulfide isomerase (EC 5.3.4.1), thiol oxidase (EC 1.8.3.2), polyphenol oxidase (EC 1.14.18.1), in particular catechol oxidase, tyrosine oxidase, and phenoloxidase, lysyl oxidase (EC 1.4.3.13), and peroxidase (EC 1.11.1.7).

In accordance with a second aspect of the present invention, there is provided a method of producing a mineral wool product which comprises the steps of contacting mineral fibres with such a binder composition, wherein the curing is carried out at temperatures from 5 to 95 °C, such as 5 to 80 °C, such as 8 to 50 °C, such as 10 to 40 °C.

The present inventors have surprisingly found that it is possible to obtain a mineral wool product comprising mineral fibres bound by a cured binder, whereby the binder composition can be produced from renewable materials to a large degree, does not contain, or contains only to a minor degree, any corrosive and/or harmful agents and the production of the mineral wool product does not lead to pollution such as VOC's (Volatile Organic Compounds) during the preparation.

## Description of the Preferred Embodiments

The mineral wool product according to the present invention as described in claim 1 comprises mineral fibres bound by a cured binder wherein the binder in its uncured state comprises:

- at least one protein,
- at least one enzyme.

In one embodiment the binder of the mineral wool product is a formaldehyde-free binder.

For the purpose of the present application, the term "formaldehyde free" is defined to characterize a mineral wool product where the emission is below 5 $\mu$g/m$^2$/h of formaldehyde from the mineral wool product, preferably below 3 $\mu$g/m$^2$/h. Preferably, the test is carried out in accordance with ISO 16000 for testing aldehyde emissions.

A surprising advantage of embodiments of mineral wool products according to the present invention is that they show self-healing properties. After being exposed to very harsh conditions when mineral wool products loose a part of their strength, the mineral wool products according to the present invention can regain a part of the original strength. This is in contrast to conventional mineral wool products for which the loss of strength after being exposed to harsh environmental conditions is irreversible. While not wanting to be bound to any particular theory, the present inventors believe that this surprising property in mineral wool products according to the present invention is due to the complex nature of the bonds formed in the network of the protein crosslinked by the enzyme which also includes quaternary structures and hydrogen bonds and allows bonds in the network to be established after returning to normal environmental conditions. For an insulation product, which when e.g. used as a roof insulation can be exposed to very high temperatures in the summer, this is an important advantage for the long term stability of the product.

In one embodiment the mineral wool product is a mineral wool insulation product, such as a mineral wool thermal or acoustical insulation product.

In one embodiment the mineral wool product is a horticultural growing media.

Protein component of the binder

The protein component of the binder is in form of one or more proteins selected from the group consisting of proteins from animal sources, including collagen, gelatine, hydrolysed gelatine, and protein from milk (casein, whey), eggs; proteins from vegetable sources, including proteins from legumes, cereals, whole grains, nuts, seeds and fruits, like protein from buckwheat, oats, rye, millet, maize (corn), rice, wheat, bulgar, sorghum, amaranth, quinoa, soybeans (soy protein), lentils, kidney beans, white beans, mung beans, chickpeas, cowpeas, lima beans, pigeon peas, lupines, wing beans, almonds, Brazil nuts, cashews, pecans, walnuts, cotton seeds, pumpkin seeds, hemp seeds, sesame seeds, and sunflower seeds; polyphenolic proteins such as mussel foot protein.

Collagen is a very abundant material in living tissue: It is the main component in connective tissue and constitutes 25-35% of the total protein content in mammals. Gelatin is derived from chemical degradation of collagen. Gelatin is water soluble and has a molecular weight of 30.000 to 300.000 g/mol dependent on the grade of hydrolysis. Gelatin is a widely used food product and it is therefore generally accepted that this compound is totally non-toxic and therefore no precautions are to be taken when handling gelatin.

The gelatin can also be further hydrolysed to smaller fragments of down to 3000 g/mol.

In a preferred embodiment, the protein component is gelatin, whereby the gelatin is preferably originating from one or more sources from the group consisting of mammal, bird species, such as from cow, pig, horse, fowl, and/or from scales, skin of fish.

In one embodiment the gelatin is a high-strength gelatin.

In one embodiment, the protein comprises polyphenolic proteins. These proteins contain a high level of a post-translationally modified-oxidized-form of tyrosine, L-3,4-dihydroxyphenylalanine (levodopa, L-DOPA). See also J. J. Wilker Nature Chem. Biol. 2011, 7, 579-580 for a reference to these proteins.

The enzyme component of the binder

The enzyme component of the binder is selected from the group consisting of transglutaminase (EC 2.3.2.13), protein disulfide isomerase (EC 5.3.4.1), thiol oxidase (EC 1.8.3.2), polyphenol oxidase (EC 1.14.18.1), in particular catechol oxidase, tyrosine oxidase, and phenoloxidase, lysyl oxidase (EC 1.4.3.13), and peroxidase (EC 1.11.1.7).

The enzymes can be both of natural sources and of recombinant sources.

In a particular preferred embodiment, the protein component is gelatine, in particular gelatine from porcine skin, in particular of medium gel strength, and the enzyme component is transglutaminase (EC 2.3.2.13).

Reaction of the binder components

The present inventors have found that some embodiments of the mineral wool product according to the present invention are best to be produced when the binder is applied to the mineral fibres under acidic conditions. Therefore, in a preferred embodiment, the binder applied to the mineral fibres comprises a pH-adjuster, in particular in form of a pH buffer.

In a preferred embodiment, the binder in its uncured state has a pH value of less than 8, such as less than 7, such as less than 6.

Other additives may be components such as one or more reactive or nonreactive silicones and may be added to the binder. Preferably, the one or more reactive or nonreactive silicone is selected from the group consisting of silicone constituted of a main chain composed of organosiloxane residues, especially diphenylsiloxane residues, alkylsiloxane residues, preferably dimethylsiloxane residues, bearing at least one hydroxyl, acyl, carboxyl or anhydride, amine, epoxy or vinyl functional group capable of reacting with at least one of the constituents of the binder composition and is preferably present in an amount of 0.1-15 weight-%, preferably from 0.1-10 weight-%, more preferably 0.3-8 weight-%, based on the total binder mass.

In one embodiment, an emulsified hydrocarbon oil may be added to the binder.

In one embodiment, an anti-fouling agent may be added to the binder.

In a preferred embodiment, the anti-fouling agent is a tannin, in particular a tannin selected from one or more components from the group consisting of tannic acid, condensed tannins (proanthocyanidins), hydrolysable tannins, gallotannins, ellagitannins, complex tannins, and/or tannin originating from one or more of oak, chestnut, staghorn sumac and fringe cups.

In one embodiment, an anti-swelling agent may be added to the binder, such as tannic acid and/or tannins.

Further additives may be additives containing calcium ions (which stabilises the transglutaminase enzyme), and antioxidants.

In one embodiment, the binder composition according to the present invention contains additives in form of linkers containing acyl groups and/or amine groups and/or thiol groups. These linkers can strengthen and/or modify the network of the cured binder.

In one embodiment, the binder compositions according to the present invention contain further additives in form of additives selected from the group consisting of PEG-type reagents, silanes, and hydroxylapatites.

Properties of the mineral wool product

In a preferred embodiment, the density of the mineral wool product is in the range of 10-900 kg/m$^3$, such as 30-800 kg/m$^3$, such as 40-600 kg/m$^3$, such as 50-250 kg/m$^3$, such as 60-200 kg/m$^3$.

In a preferred embodiment, the mineral wool product according to the present invention is an insulation product, in particular having a density of 10 to 200 kg/m$^3$.

In a preferred embodiment, the loss on ignition (LOI) of the mineral wool product according to the present invention is within the range of 0.1 to 25.0 %, such as 0.3 to 18.0 %, such as 0.5 to 12.0 %, such as 0.7 to 8.0 % by weight.

Method of producing a mineral wool product

The present invention also provides a method as described in claim 7 for producing a mineral wool product by binding mineral fibres with the binder composition.

A particular advantage of the mineral wool product according to the present invention is that it does not require high temperatures for curing. This does not only save energy, reduces VOC and obviates the need for machinery to be highly temperature resistant, but also allows for a high flexibility in a process for the production of mineral wool products with these binders.

In one embodiment the method comprises the steps of:

- making a melt of raw materials,
- fibrerising the melt by means of a fiber forming apparatus to form mineral fibres,
- providing the mineral fibres in the form of a collected web,
- mixing the binder with the mineral fibres before, during or after the provision of the collected web to form a mixture of mineral fibres and binder,
- curing the mixture of mineral fibres and binder.

In one embodiment, the binder is supplied in the close vicinity of the fibre forming apparatus, such as a cup spinning apparatus or a cascade spinning apparatus, in either case immediately after the fibre formation. The fibres with applied binder are thereafter conveyed onto a conveyor belt as a web.

The web may be subjected to longitudinal or length compression after the fibre formation and before substantial curing has taken place.

Fiber forming apparatus

There are various types of centrifugal spinners for fiberising mineral melts.

A conventional centrifugal spinner is a cascade spinner which comprises a sequence of a top (or first) rotor and a subsequent (or second) rotor and optionally other subsequent rotors (such as third and fourth rotors). Each rotor rotates about a different substantially horizontal axis with a rotational direction opposite to the rotational direction of the or each adjacent rotor in the sequence. The different horizontal axes are arranged such that melt which is poured on to the top rotor is thrown in sequence on to the peripheral surface of the or each subsequent rotor, and fibres are thrown off the or each subsequent rotor, and optionally also off the top rotor.

In one embodiment, a cascade spinner or other spinner is arranged to fiberise the melt and the fibres are entrained in air as a cloud of the fibres.

Many fiber forming apparatuses comprise a disc or cup that spins around a substantially vertical axis. It is then conventional to arrange several of these spinners in-line, i.e. substantially in the first direction, for instance as described in GB-A-926,749, US-A-3,824,086 and WO-A-83/03092.

There is usually a stream of air associated with the one or each fiberising rotor whereby the fibres are entrained in this air as they are formed off the surface of the rotor.

In one embodiment, binder and/or additives is added to the cloud of fibres by known means. The amount of binder and/or additive may be the same for each spinner or it may be different.

In one embodiment, a hydrocarbon oil may be added into the cloud of fibres.

As used herein, the term "collected web" is intended to include any mineral fibres that have been collected together on a surface, i.e. they are no longer entrained in air, e.g. the fibrerised mineral fibres, granulate, tufts or recycled web waste. The collected web could be a primary web that has been formed by collection of fibres on a conveyor belt and provided as a starting material without having been cross-lapped or otherwise consolidated.

Alternatively, the collected web could be a secondary web that has been formed by crosslapping or otherwise consolidating a primary web. Preferably, the collected web is a primary web.

In one embodiment the mixing of the binder with the mineral fibres is done after the provision of the collected web in the following steps:

- subjecting the collected web of mineral fibres to a disentanglement process,
- suspending the mineral fibres in a primary air flow,
- mixing binder composition with the mineral fibres before, during or after the disentanglement process to form a mixture of mineral fibres and binder.

A method of producing a mineral wool product comprising the process step of disentanglement is described in EP10190521.

In one embodiment, the disentanglement process comprises feeding the collected web of mineral fibres from a duct with a lower relative air flow to a duct with a higher relative air flow. In this embodiment, the disentanglement is believed to occur, because the fibres that enter the duct with the higher relative air flow first are dragged away from the subsequent fibres in the web. This type of disentanglement is particularly effective for producing open tufts of fibres, rather than the compacted lumps that can result in an uneven distribution of materials in the product.

According to a particularly preferred embodiment, the disentanglement process comprises feeding the collected web to at least one roller which rotates about its longitudinal axis and has spikes protruding from its circumferential surface. In this embodiment, the rotating roller will usually also contribute at least in part to the higher relative air flow. Often, rotation of the roller is the sole source of the higher relative air flow.

In preferred embodiments, the mineral fibres and optionally the binder are fed to the roller from above. It is also preferred for the disentangled mineral fibres and optionally the binder to be thrown away from the roller laterally from the lower part of its circumference. In the most preferred embodiment, the mineral fibres are carried approximately 180 degrees by the roller before being thrown off.

The binder may be mixed with the mineral fibres before, during or after the disentanglement process. In some embodiments, it is preferred to mix the binder with the fibres prior to the disentanglement process. In particular, the fibres can be in the form of an uncured collected web containing binder.

It is also feasible that the binder be pre-mixed with a collected web of mineral fibres before the disentanglement process. Further mixing could occur during and after the disentanglement process. Alternatively, it could be supplied to the primary air flow separately and mixed in the primary air flow.

The mixture of mineral fibres and binder is collected from the primary air flow by any suitable means. In one embodiment, the primary air flow is directed into the top of a cyclone chamber, which is open at its lower end and the mixture is collected from the lower end of the cyclone chamber.

The mixture of mineral fibres and binder is preferably thrown from the disentanglement process into a forming chamber.

Having undergone the disentanglement process, the mixture of mineral fibres and binder is collected, pressed and cured. Preferably, the mixture is collected on a foraminous conveyor belt having suction means positioned below it.

In a preferred method according to the invention, the mixture of binder and mineral fibres, having been collected, is pressed and cured.

In a preferred method according to the invention, the mixture of binder and mineral fibres, having been collected, is scalped before being pressed and cured.

The method may be performed as a batch process, however according to an embodiment the method is performed at a mineral wool production line feeding a primary or secondary mineral wool web into the fibre separating process, which provides a particularly cost efficient and versatile method to provide composites having favourable mechanical properties and thermal insulation properties in a wide range of densities.

At the same time, because of the curing at ambient temperature, the likelihood of uncured binder spots is strongly decreased.

Curing

The web is cured by a chemical and/or physical reaction of the binder components.

In one embodiment, the curing takes place in a curing device.

The curing is carried out at temperatures from 5 to 95 °C, such as 5 to 80 °C, such as 5 to 60 °C, such as 8 to 50 °C, such as 10 to 40 °C.

In one embodiment the curing takes place in a conventional curing oven for mineral wool production operating at a temperature of from 5 to 95 °C, such as 5 to 80 °C, such as 10 to 60 °C, such as 20 to 40 °C.

The curing process may commence immediately after application of the binder to the fibres. The curing is defined as a process whereby the binder composition undergoes a chemical reaction which usually increases the molecular weight of the compounds in the binder composition and thereby increases the viscosity of the binder composition, usually until the binder composition reaches a solid state.

In one embodiment the curing process comprises cross-linking and/or water inclusion as crystal water.

In one embodiment the cured binder contains crystal water that may decrease in content and raise in content depending on the prevailing conditions of temperature, pressure and humidity.

In one embodiment the curing process comprises a drying process.

In a preferred embodiment, the curing of the binder in contact with the mineral fibers takes place in a heat press.

The curing of a binder in contact with the mineral fibers in a heat press has the particular advantage that it enables the production of high-density products.

In one embodiment the curing process comprises drying by pressure. The pressure may be applied by blowing air or gas to the mixture of mineral fibres and binder. The blowing process may be accompanied by heating or cooling or it may be at ambient temperature.

In one embodiment the curing process takes place in a humid environment.

The humid environment may have a relative humidity RH of 60-99%, such as 70-95%, such as 80-92%. The curing in a humid environment may be followed by curing or drying to obtain a state of the prevalent humidity.

In one embodiment the curing is performed in oxygen-depleted surroundings.

Without wanting to be bound by any particular theory, the applicant believes that performing the curing in an oxygen-depleted surrounding increases the stability of the enzyme component in some embodiments, in particular of the transglutaminase enzyme, and thereby improves the crosslinking efficiency. In one embodiment, the curing process is therefore performed in an inert atmosphere, in particular in an atmosphere of an inert gas, like nitrogen.

The mineral wool product can be in any conventional configuration, for instance a mat or slab, and can be cut and/or shaped (e.g. into pipe sections) before, during or after curing of the binder.

## Advantages of the binder composition

The mineral wool product according to the present invention has the surprising advantage that it can be produced by a very simple binder which requires as little as only two components, namely at least one protein and at least one enzyme, whereby no pre-reaction of this binder is necessary. The mineral wool product according to the present invention is therefore produced from natural and non-toxic components and is therefore safe to work with. At the same time, the mineral wool product according to the present invention is produced from a binder based on renewable resources.

Because the binder used for the production of the mineral wool product according to the present invention can be cured at ambient temperature or in the vicinity of ambient temperature, the energy consumption during the production of the products is very low. The non-toxic and non-corrosive nature of embodiments of the binders in combination with the curing at ambient temperatures allows a much less complex machinery to be involved. At the same time, because of the curing at ambient temperature, the likelihood of uncured binder spots is strongly decreased.

Further important advantages are the self-repair capacities of mineral wool products produced from the binders.

A further advantage of the mineral wool products is that they may be shaped as desired after application of the binder but prior to curing. This opens the possibility for making tailor-made products, like pipe sections.

A further advantage is the strongly reduced punking risk.

Punking may be associated with exothermic reactions during manufacturing of the mineral wool product which increase temperatures through the thickness of the insulation causing a fusing or devitrification of the mineral fibres and eventually creating a fire hazard. In the worst case, punking causes fires in the stacked pallets stored in warehouses or during transportation.

Yet another advantage is the absence of emissions during curing, in particular the absence of VOC emissions.

## Examples

In the following examples, several binders which fall under the definition of the present invention were prepared and compared to binders according to the prior art.

## Binders according to the prior art

The following properties were determined for the binders according the prior art.

*Reagents*

50% aq. hypophosphorous acid and 28% aq. ammonia were supplied by Sigma Aldrich. D-(+)-glucose monohydrate was supplied by Merck. 75.1 % aq. glucose syrup with a DE-value of 95 to less than 100 (C*sweet D 02767 ex Cargill) was supplied by Cargill. Silane (Momentive VS-142) was supplied by Momentive and was calculated as 100% for simplicity. All other components were supplied in high purity by Sigma-Aldrich and were assumed anhydrous for simplicity.

*Binder component solids content - definition*

The content of each of the components in a given binder solution before curing is based on the anhydrous mass of the components. The following formula can be used:

$$Binder\ component\ solids\ content\ (\%) = \frac{binder\ component\ A\ solids\ (g) + binder\ component\ B\ solids\ (g) + \cdots}{total\ weight\ of\ mixture\ (g)} \times 100\%$$

*Binder solids - definition and procedure*

The content of binder after curing is termed "binder solids".
Disc-shaped stone wool samples (diameter: 5 cm; height 1 cm) were cut out of stone wool and heat-treated at 580 °C for at least 30 minutes to remove all organics. The solids of the binder mixture (see below for mixing examples) were measured by distributing a sample of the binder mixture (approx. 2 g) onto a heat treated stone wool disc in a tin foil container. The weight of the tin foil container containing the stone wool disc was weighed before and directly after addition of the binder mixture. Two such binder mixture loaded stone wool discs in tin foil containers were produced and they were then heated at 200 °C for 1 hour. After cooling and storing at room temperature for 10 minutes, the samples were weighed and the binder solids was calculated as an average of the two results. A binder with the desired binder solids could then be produced by diluting with the required amount of water and 10% aq. silane (Momentive VS-142).

*Reaction loss - definition*

The reaction loss is defined as the difference between the binder component solids content and the binder solids.

*Mechanical strength studies (tablet tests) - procedure*

The mechanical strength of the binders was tested in a tablet test. For each binder, six tablets were manufactured from a mixture of the binder and stone wool shots from the stone wool spinning production. The shots are particles which have the same melt composition as the stone wool fibres, and the shots are normally considered a waste product from the spinning process. The shots used for the tablet composition have a size of 0.25-0.50 mm.
A 15% binder solids binder solution containing 0.5% silane (Momentive VS-142) of binder solids was obtained as described above under "binder solids". A sample of this binder solution (4.0 g) was mixed well with shots (20.0 g). The resulting mixture was then transferred into a round aluminum foil container (bottom Ø = 4.5 cm, top Ø = 7.5 cm, height = 1.5 cm). The mixture was then pressed hard with a suitably sized flat bottom glass or plastic beaker to generate an even tablet surface. Six tablets from each binder were made in this fashion. The resulting tablets were then cured at 250 °C for 1 h. After cooling to room temperature, the tablets were carefully taken out of the containers. Three of the tablets were aged in a water bath at 80 °C for 3 h.
After drying for 1-2 days, all tablets were then broken in a 3 point bending test (test speed: 10.0 mm/min; rupture level: 50%; nominal strength: 30N/mm$^2$; support distance: 40 mm; max deflection 20 mm; nominal e-module 10000 N/mm$^2$) on a Bent Tram machine to investigate their mechanical strengths. The tablets were placed with the "bottom face" up (i.e. the face with Ø = 4.5 cm) in the machine.

*Theoretical binder content in tablets - definition*

The theoretical binder content in tablets is calculated by dividing the binder solids used in the tablet production with the sum of the binder solids and shots used in the tablet production. The following formula can be used:

$$Theoretical\ binder\ content\ (\%) = \frac{binder\ mixture\ used\ (g) \times binder\ solids\ (\%)}{binder\ mixture\ used\ (g) \times binder\ solids\ (\%) + shots\ used\ (g)} \times 100\%$$

For each binder mixture, six tablets were produced. The theoretical binder content for each binder tested was calculated as an average of these six tablets.

Reference binders from the prior art prepared as comparative examples

*Binder example, reference binder A*

A mixture of anhydrous citric acid (10.2 g, 53.1 mmol) and D-(+)-glucose monohydrate (57.3 g; thus efficiently 52.1 g dextrose) in water (157.5 g) was stirred at room temperature until a clear solution was obtained. 28% aq. ammonia (7.80 g; thus efficiently 2.16 g, 128.4 mmol ammonia) was then added dropwise. The binder solids were then measured (17.4%). For mechanical strength studies (15% binder solids solution, 0.5% silane of binder solids), the binder mixture was diluted with water (0.149 g / g binder mixture) and 10% aq. silane (0.009 g / g binder mixture, Momentive VS-142). The final binder mixture had pH = 5.1.

*Binder example, reference binder B (phenol-formaldehyde resin modified with urea, a PUF-resol)*

A phenol-formaldehyde resin is prepared by reacting 37% aq. formaldehyde (606 g) and phenol (189 g) in the presence of 46% aq. potassium hydroxide (25.5 g) at a reaction temperature of 84°C preceded by a heating rate of approximately 1°C per minute. The reaction is continued at 84 °C until the acid tolerance of the resin is 4 and most of the phenol is converted. Urea (241 g) is then added and the mixture is cooled.

The acid tolerance (AT) expresses the number of times a given volume of a binder can be diluted with acid without the mixture becoming cloudy (the binder precipitates). Sulfuric acid is used to determine the stop criterion in a binder production and an acid tolerance lower than 4 indicates the end of the binder reaction. To measure the AT, a titrant is produced from diluting 2.5 ml conc. sulfuric acid (>99 %) with 1 L ion exchanged water. 5 mL of the binder to be investigated is then titrated at room temperature with this titrant while keeping the binder in motion by manually shaking it; if preferred, use a magnetic stirrer and a magnetic stick. Titration is continued until a slight cloud appears in the binder, which does not disappear when the binder is shaken.

The acid tolerance (AT) is calculated by dividing the amount of acid used for the titration (mL) with the amount of sample (mL):

$$AT = (Used\ titration\ volume\ (mL)) / (Sample\ volume\ (mL))$$

Using the urea-modified phenol-formaldehyde resin obtained, a binder is made by addition of 25% aq. ammonia (90 mL) and ammonium sulfate (13.2 g) followed by water (1.30 kg). The binder solids were then measured as described above and the mixture was diluted with the required amount of water and silane (Momentive VS-142) for mechanical strength studies (15% binder solids solution, 0.5% silane of binder solids).

*Binder example, reference binder C*

A mixture of L-ascorbic acid (1.50 g, 8.52 mmol) and 75.1% aq. glucose syrup (18.0 g; thus efficiently 13.5 g glucose syrup) in water (30.5 g) was stirred at room temperature until a clear solution was obtained. 50% aq. hypophosphorous acid (0.60 g; thus efficiently 0.30 g, 4.55 mmol hypophosphorous acid) and urea (0.75 g) were then added. 28% aq. ammonia (0.99 g; thus efficiently 0.28 g, 16.3 mmol ammonia) was then added dropwise until pH = 6.9. The binder solids were then measured (21.5%). For mechanical strength studies (15% binder solids solution, 0.5% silane of binder solids), the binder mixture was diluted with water (0.423 g / g binder mixture) and 10% aq. silane (0.011 g / g binder mixture, Momentive VS-142). The final binder mixture had pH = 7.0.

*Binder example, reference binder D*

A mixture of 75.1% aq. glucose syrup (60.0 g; thus efficiently 45.0 g glucose syrup), ammonium sulfamate (2.25 g, 19.7 mmol) and urea (2.25 g) in water (105.1 g) was stirred at room temperature until a clear solution was obtained. 28% aq. ammonia (0.12 g; thus efficiently 0.03 g, 1.97 mmol ammonia) was then added dropwise until pH = 8.2. The binder solids were then measured (21.6%). For mechanical strength studies (15% binder solids solution, 0.5% silane of binder solids), the binder mixture was diluted with water (0.432 g / g binder mixture) and 10% aq. silane (0.011 g / g binder mixture, Momentive VS-142). The final binder mixture had pH = 8.2.

*Binder example, reference binder E (based on alkanolamine-polycarboxylic acid anhydride reaction products)*

Diethanolamine (DEA, 231.4 g) is placed in a 5-litre glass reactor provided with a stirrer and a heating/cooling jacket. The temperature of the diethanolamine is raised to 60 °C where after tetrahydrophthalic anhydride (THPA, 128.9 g) is added. After raising the temperature and keeping it at 130 °C, a second portion of tetrahydrophthalic anhydride (64.5 g) is added followed by trimellitic anhydride (TMA, 128.9 g). After reacting at 130 °C for 1 hour, the mixture is cooled to 95 °C. Water (190.8 g) is added and stirring is continued for 1 hour. After cooling to ambient temperature, the mixture is poured into water (3.40 kg) and 50% aq. hypophosphorous acid (9.6 g) and 25% aq. ammonia (107.9 g) are added under stirring. Glucose syrup (1.11 kg) is heated to 60 °C and then added under stirring followed by 50% aq. silane (5.0 g, Momentive VS-142). The binder solids were then measured as described above and the mixture was diluted with the required amount of water for mechanical strength measurements (15% binder solids solutions).

*Binder example, reference binder F (based on alkanolamine-polycarboxylic acid anhydride reaction products)*

Diethanolamine (DEA, 120.5 g) is placed in a 5-litre glass reactor provided with a stirrer and a heating/cooling jacket. The temperature of the diethanolamine is raised to 60 °C where after tetrahydrophthalic anhydride (THPA, 67.1 g) is added. After raising the temperature and keeping it at 130 °C, a second portion of tetrahydrophthalic anhydride (33.6 g) is added followed by trimellitic anhydride (TMA, 67.1 g). After reacting at 130 °C for 1 hour, the mixture is cooled to 95 °C. Water (241.7 g) is added and stirring is continued for 1 hour. Urea (216.1 g) is then added and stirring is continued until all solids are dissolved. After cooling to ambient temperature, the mixture is poured into water (3.32 kg) and 50% aq. hypophosphorous acid (5.0 g) and 25% aq. ammonia (56.3 g) are added under stirring. Glucose syrup (1.24 kg) is heated to 60 °C and then added under stirring followed by 50% aq. silane (5.0 g, Momentive VS-142). The binder solids were then measured as described above and the mixture was diluted with the required amount of water for mechanical strength measurements (15% binder solids solutions).

Binders according to the present invention

The following properties were determined for the binders according the present invention.

*Reagents*

Medium gel strength gelatin from porcine skin (170-195 g Bloom) and sodium hydroxide were obtained from Sigma-Aldrich. TI Transglutaminase formula was obtained from Modernist Pantry. For simplicity, these reagents were considered completely pure and anhydrous.

*Binder component solids content - definition*

The content of each of the components in a given binder solution before curing is based on the anhydrous mass of the components. The following formula can be used:

$$Binder\ component\ solids\ content\ (\%) = \frac{binder\ component\ A\ solids\ (g) + binder\ component\ B\ solids\ (g) + \cdots}{total\ weight\ of\ mixture\ (g)} \times 100\%$$

The binder examples listed below were mixed to a binder component solids content of 15±1.5% for convenience.

*Mechanical strength studies (tablet tests) - procedure*

The mechanical strength of the binders was tested in a tablet test. For each binder, six tablets were manufactured from a mixture of the binder and stone wool shots from the stone wool spinning production. The shots are particles which have the same melt composition as the stone wool fibers, and the shots are normally considered a waste product from the spinning process. The shots used for the tablet composition have a size of 0.25-0.50 mm.

A binder mixture containing 15±1.5% binder component solids was obtained as described in the examples below. A sample of this binder solution (4.0 g) was mixed well with shots (20.0 g). The resulting mixture was then transferred into a round aluminum foil container (bottom Ø = 4.5 cm, top Ø = 7.5 cm, height = 1.5 cm). The mixture was spread out evenly in the container using a broad spatula, generating an even tablet surface. Six tablets from each binder were made in this fashion.

Containers with tablets made with binders that were intended to cure at room temperature were left at room temperature for two days. Containers with tablets that were intended to cure below room temperature were first left at 11 °C for one day followed by two days at room temperature. Containers with tablets that were intended to cure above room temperature were left at 41 °C, 61 °C or 81 °C for 5 h, 2 h, or 1 h, respectively, followed by one day at room temperature.

The tablets were then carefully taken out of the containers. Three of the tablets were aged in a water bath at 80 °C for 3 h.

After drying for two days, all tablets were then broken in a 3 point bending test (test speed: 10.0 mm/min; rupture level: 50%; nominal strength: 30 N/mm$^2$; support distance: 40 mm; max deflection 20 mm; nominal e-module 10000 N/mm$^2$) on a Bent Tram machine to investigate their mechanical strengths. The tablets were placed with the "bottom face" up (i.e. the face with Ø = 4.5 cm) in the machine.

*Theoretical binder content in tablets - definition*

The theoretical binder content in tablets is calculated by dividing the binder component solids used in the tablet production with the sum of the binder component solids and shots used in the tablet production. The following formula can be used:

$$Theoretical\ binder\ content\ (\%) = \frac{binder\ mixture\ used\ (g) \times binder\ comp.\ solids\ content\ (\%)}{binder\ mixture\ used\ (g) \times binder\ comp.\ solids\ content\ (\%) + shots\ used\ (g)} \times 100\%$$

For each binder mixture, six tablets were produced. The theoretical binder content for each binder tested was calculated as an average of these six tablets.

*Measured binder content in tablets - definition*

The measured binder content in tablets is calculated by dividing the weight of the dried tablet minus the shots in the tablet with the weight of the dried tablet. The following calculation can be used:

$$Meas.\ binder\ content\ (\%) = \frac{dry\ tablet\ (g) - freshly\ made\ tablet\ (g) \times \dfrac{shots\ used\ (g)}{shots\ used\ (g) + binder\ mixture\ used\ (g)}}{dry\ tablet\ (g)} \times 100\%$$

For each binder mixture, six tablets were produced. The measured binder content for each binder tested was calculated as an average of these six tablets.

*Film tests - procedure*

A binder mixture containing 15±1.5% binder component solids was obtained as described in the examples below. A sample large enough to cover the bottom with a layer of approx. 1-2 mm thickness was then transferred into a round aluminum foil container (bottom Ø = 4.5 cm, top Ø = 7.5 cm, height = 1.5 cm). For each binder mixture, two such samples were produced and they were then left at room temperature for two days.

The generated films were carefully taken out of the container and one of the films was tested for water resistance by submerging the film halfway into water at 80 °C. Binder compositions with no water resistance would dissolve quickly in this test.

Binder compositions according to the present invention

*Binder example, entry (this example does not fall within the scope of the invention)*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.1). The solution was stirred for 30 minutes further at 50 °C before being used in the subsequent experiments.

*Binder example, entry (this example does not fall within the scope of the invention)*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.1). 1M NaOH (3.10 g) was then added (pH 8.8) and the resulting solution was stirred for 30 minutes further at 50 °C before being used in the subsequent experiments.

*Binder example, entry 6*

Gelatin from porcine skin, medium gel strength (10.0 g) was swelled in water (51.7 g) for 30 min at room temperature. The mixture was then placed in a water bath at 50 °C and stirred a few minutes until a clear solution was obtained (pH 5.2). The solution was stirred for 30 minutes at 50 °C and a solution of TI transglutaminase (0.20 g) in water (5.0 g) was then added (pH 5.2). The resulting mixture was stirred for a few minutes further at 50 °C before being used in the subsequent experiments.

*Binder example, entry 7*

Gelatin from porcine skin, medium gel strength (10.0 g) was swelled in water (51.7 g) for 30 min at room temperature. The mixture was then placed in a water bath at 50 °C and stirred a few minutes until a clear solution was obtained (pH 5.1). The solution was stirred for 30 minutes at 50 °C and a solution of TI transglutaminase (0.50 g) in water (5.0 g) was then added (pH 5.2). The resulting mixture was stirred for a few minutes further at 50 °C before being used in the subsequent experiments.

*Binder example, entry 8*

Gelatin from porcine skin, medium gel strength (10.0 g) was swelled in water (51.7 g) for 30 min at room temperature. The mixture was then placed in a water bath at 50 °C and stirred a few minutes until a clear solution was obtained (pH 5.3). The solution was stirred for 30 minutes at 50 °C and a solution of TI transglutaminase (1.00 g) in water (5.0 g) was then added (pH 5.3). The resulting mixture was stirred for a few minutes further at 50 °C before being used in the subsequent experiments.

The other binders mentioned in Table 1 were prepared in a manner analogous to the preparations described above.

TABLE 1-1

| | Reference binders | | | | | |
|---|---|---|---|---|---|---|
| **Example** | A | B | C | D | E | F |
| **Binder composition** | | | | | | |
| Acid or Ascorb. acid (%-wt.) | | | | | | |
| L-Ascorbic acid | - | - | 10 | - | - | - |
| Citric acid | 16 | - | - | - | - | - |
| Carbohydrate (%-wt.) | | | | | | |
| Glucose syrup | | - | 90 | 100 | - | - |
| Glucose | 84 | - | - | - | - | - |
| Additive (%-wt.)[a] | | | | | | |
| Hypophosphorous acid | - | - | 2 | - | - | - |
| Ammonium sulfamate | - | - | - | 5 | - | - |
| Urea | - | - | 5 | 5 | - | - |
| Amine (equiv.) [b] | | | | | | |
| Ammonia (added) | 0.8 | - | 1.2 | 0.1 | - | - |
| Silane (% of binder solids) | 0.5 | 0.5 | 0.5 | 0.5 | - | - |

(continued)

|  | Reference binders | | | | | |
| Example | A | B | C | D | E | F |
| **Binder composition** | | | | | | |
| | | | | | | |
| **Binder properties** | | | | | | |
| Binder component solids content (%) | - | - | 21.8 | 20.2 | 21.1 | 21.6 |
| Reaction loss (%) | 37.3 | 28.5 | 31.1 | 25.9 | 28.9 | 30.6 |
| Binder solids (%) | 15.0 | 15.0 | 15.0 | 15.0 | 15.0 | 15.0 |
| pH of binder mixture | 5.1 | 9.6 | 7.0 | 8.2 | 6.1 | 6.2 |
| | | | | | | |
| **Curing conditions** | | | | | | |
| Temperature (°C) | 250 | 250 | 250 | 250 | 250 | 250 |
| | | | | | | |
| **Tablet properties** | | | | | | |
| Theoretical binder content in tablets (%)[c] | 2.9 | 2.9 | 2.9 | 2.9 | 2.9 | 2.9 |
| Mechanical strength, unaged (kN) | 0.17 | 0.16 | 0.16 | 0.16 | 0.20 | 0.09 |
| Mechanical strength, aged (kN) | 0.13 | 0.09 | 0.08 | 0.09 | 0.16 | 0.07 |
| [a] Of Carbohydrate + ascorbic acid. [b] Molar amine equivalents relative to molar mineral or organic acid equivalents. [c] Based on binder solids. | | | | | | |

TABLE 1-2

|  | Gelatin, | various conditions | (no | transglutaminase) | |
| Example | 1 | 2 | 3 | 4 | 5 |
| **Binder composition** | | | | | |
| Protein (%-wt.) | | | | | |
| Gelatin (porcine skin), medium gel strength | 100 | 100 | 100 | 100 | 100 |
| Enzyme mixture (%-wt.) [a] | | | | | |
| TI Transalutaminase | - | - | - | - | - |
| Base (%-wt.) [b] | | | | | |
| Sodium hydroxide | - | - | - | - | 1.2 |
| | | | | | |
| **Binder properties** | | | | | |
| Binder component solids content (%) | 15.0 | 15.0 | 15.0 | 15.0 | 14.5 |
| pH of binder mixture | 5.1 | 5.0 | 5.1 | 5.0 | 8.8 |
| | | | | | |
| **Curing conditions** | | | | | |
| Temperature (°C) | rt | 43 | 61 | 81 | rt |
| | | | | | |

(continued)

| Tablet properties | | | | | |
|---|---|---|---|---|---|
| Theoretical binder content in tablets (%)[c] | 2.9 | 2.9 | 2.9 | 2.9 | 2.8 |
| Measured binder content in tablets (%) | 3.9 | 4.1 | 4.0 | 3.9 | 3.8 |
| Mechanical strength, unaged (kN) | 0.24 | 0.29 | 0.28 | 0.29 | 0.23 |
| Mechanical strength, aged (kN) | 0[d] | 0[d] | 0[d] | 0[d] | 0[d] |
| | | | | | |
| Film properties | | | | | |
| Stable in ageing test | No | - | - | - | No |
| [a] Of gelatin. [b] Of gelatin + transglutaminase. [c] Based on binder component solids content. [d] Complete disintegration of the tablets was observed during the ageing treatment. | | | | | |

TABLE 1-3

| | Gelatin, transglutaminase, rt | | | Gelatin, transglutaminase, other temp. | | |
|---|---|---|---|---|---|---|
| Example | 6 | 7 | 8 | 9 | 10 | 11 |
| **Binder composition** | | | | | | |
| Protein (%-wt.) | | | | | | |
| Gelatin (porcine skin), medium gel strength | 100 | 100 | 100 | 100 | 100 | 100 |
| Enzyme mixture (%-wt.) [a] | | | | | | |
| TI Transalutaminase | 2 | 5 | 10 | 5 | 5 | 5 |
| Base (%-wt.) [b] | | | | | | |
| Sodium hydroxide | - | - | - | - | - | - |
| | | | | | | |
| **Binder properties** | | | | | | |
| Binder component solids content (%) | 15.3 | 15.6 | 16.3 | 15.6 | 15.6 | 15.6 |
| pH of binder mixture | 5.2 | 5.2 | 5.3 | 5.0 | 5.1 | 5.1 |
| | | | | | | |
| **Curing conditions** | | | | | | |
| Temperature (°C) | rt | rt | rt | 11 | 43 | 61 |
| | | | | | | |
| **Tablet properties** | | | | | | |
| Theoretical binder content in tablets (%)[c] | 3.0 | 3.0 | 3.1 | 3.0 | 3.0 | 3.0 |
| Measured binder content in tablets (%) | 4.1 | 4.1 | 4.4 | 4.1 | 4.0 | 4.0 |
| Mechanical strength, unaged (kN) | 0.28 | 0.31 | 0.26 | 0.32 | 0.26 | 0.23 |
| Mechanical strength, aged (kN) | 0.10 | 0.22 | 0.22 | 0.19 | 0.18 | 0.12 |
| | | | | | | |
| **Film properties** | | | | | | |

(continued)

| Film properties | | | | | | |
|---|---|---|---|---|---|---|
| Stable in ageing test | - | Yes | - | - | - | - |
| [a] Of gelatin. [b] Of gelatin + transglutaminase. [c] Based on binder component solids content. | | | | | | |

The following observations and conclusions can be obtained from the experimental work documented in Tables 1-1 to 1-3:

When comparing the theoretical binder content and the measured binding content for the examples according to the present invention in Table 1-3, it can be seen that the measured binder content is considerably higher. The applicant believes that this is in part due to the inclusion of considerable amounts of crystal water in the cured binder. Further, due to the curing at very low temperatures, the binders according to the present invention do not experience any significant reaction loss. Accordingly, a higher LOI and thereby a higher binder content, can be achieved with the use of less organic starting material, when compared with other binders based on renewable resources like the reference binders A, C and D.

As can be seen when comparing the results documented in Table 1-3 with the results documented in Table 1-1, the mineral wool products according to the present invention can have a higher mechanical strength (aged and unaged) by using the same amount of organic starting material for the binder (theoretical binder content) and a much lower curing temperature. This allows a better product to be obtained while at the same time the energy consumption is decreased and a simpler equipment can be used.

## Claims

1. A mineral wool product comprising mineral fibres bound by a cured binder wherein the binder in its uncured state comprises:

   - at least one protein,
   - at least one enzyme,
   wherein the at least one protein is selected from the group consisting of proteins from animal sources, including collagen, gelatine, hydrolysed gelatine, and protein from milk (casein, whey), eggs; proteins from vegetable sources, including proteins from legumes, cereals, whole grains, nuts, seeds and fruits, like protein from buckwheat, oats, rye, millet, maize (corn), rice, wheat, bulgar, sorghum, amaranth, quinoa, soybeans (soy protein), lentils, kidney beans, white beans, mung beans, chickpeas, cowpeas, lima beans, pigeon peas, lupines, wing beans, almonds, Brazil nuts, cashews, pecans, walnuts, cotton seeds, pumpkin seeds, hemp seeds, sesame seeds, and sunflower seeds; polyphenolic proteins such as mussel foot protein,
   wherein the at least one enzyme is selected from the group consisting of transglutaminase (EC 2.3.2.13), protein disulfide isomerase (EC 5.3.4.1), thiol oxidase (EC 1.8.3.2), polyphenol oxidase (EC 1.14.18.1), in particular catechol oxidase, tyrosine oxidase, and phenoloxidase, lysyl oxidase (EC 1.4.3.13), and peroxidase (EC 1.11.1.7).

2. A mineral wool product according to claim 1, wherein the collagen or gelatin is originating from one or more sources from the group consisting of mammal, bird species, such as from cow, pig, horse, fowl, and/or from scales, skin of fish.

3. A mineral wool product according to any of the above claims, wherein the binder in its uncured state comprises a pH adjuster, in particular in form of a pH buffer.

4. A mineral wool product according to any of the above claims, wherein the binder in its uncured state has a pH value less than 8, such as less than 7, such as less than 6.

5. A mineral wool product according to any of the above claims, wherein the density of the mineral wool product is in the range of 10-900 $kg/m^3$, such as 30-800 $kg/m^3$, such as 40-600 $kg/m^3$, such as 50-250 $kg/m^3$, such as 60-200 $kg/m^3$.

6. A mineral wool product according to any of the above claims, wherein the loss on ignition (LOI) is within the range of 0.1 to 25.0 %, such as 0.3 to 18.0 %, such as 0.5 to 12.0 %, such as 0.7 to 8.0 % by weight.

7. A method of producing a mineral wool product which comprises the steps of contacting mineral fibres with a binder according to any one of the claims 1 to 6, and curing the binder, wherein the curing is carried out at temperatures from 5 to 95 °C, such as 5 to 80 °C, such as 8 to 50 °C, such as 10 to 40 °C.

8. A method of producing a mineral wool product according to claim 7, wherein the method comprises the steps of:

   - making a melt of raw materials,
   - fibrerising the melt by means of a fiber forming apparatus to form mineral fibres,
   - providing the mineral fibres in the form of a collected web,
   - mixing the binder with the mineral fibres before, during or after the provision of the collected web to form a mixture of mineral fibres and binder,
   - curing the mixture of mineral fibres and binder.

9. A method of producing a mineral wool product according to claim 8, wherein the mixing of the binder with the mineral fibres is done after the provision of the collected web in the following steps:

   - subjecting the collected web of mineral fibres to a disentanglement process,
   - suspending the mineral fibres in a primary air flow,
   - mixing the binder with the mineral fibres before, during or after the disentanglement process to form a mixture of mineral fibres and binder.

10. A method of producing a mineral wool product according to claim 7-9, wherein the curing process comprises a drying process, in particular by blowing air or gas over the mineral wool product and/or or by increasing temperature.

11. A method of producing a mineral wool product according to claim 7-10, wherein the curing is performed in oxygen-depleted surroundings.

**Patentansprüche**

1. Mineralwollprodukt, umfassend Mineralfasern, die durch ein gehärtetes Bindemittel gebunden sind, wobei das Bindemittel in seinem ungehärteten Zustand umfasst:

   - mindestens ein Protein,
   - mindestens ein Enzym,
   wobei das mindestens eine Protein ausgewählt ist aus der Gruppe bestehend aus Proteinen aus tierischen Quellen, einschließlich Kollagen, Gelatine, hydrolysierter Gelatine und Protein aus Milch (Kasein, Molke), Eiern; Proteinen aus pflanzlichen Quellen, einschließlich Proteinen aus Hülsenfrüchten, Getreide, Vollkorn, Nüssen, Samen und Früchten, wie Protein aus Buchweizen, Hafer, Roggen, Hirse, Mais (Korn), Reis, Weizen, Bulgar, Sorghum, Amaranth, Quinoa, Sojabohnen (Sojaprotein), Linsen, Kidneybohnen, weißen Bohnen, Mungobohnen, Kichererbsen, Kuherbsen, Limabohnen, Taubenerbsen, Lupinen, Flügelbohnen, Mandeln, Paranüssen, Cashewnüssen, Pekannüssen, Walnüssen, Baumwollsamen, Kürbiskernen, Hanfsamen, Sesamsamen und Sonnenblumenkernen; polyphenolischen Proteinen, wie z.B. Muschelfußprotein,
   wobei das mindestens eine Enzym ausgewählt ist aus der Gruppe bestehend aus Transglutaminase (EC 2.3.2.13), Proteindisulfidisomerase (EC 5.3.4.1), Thioloxidase (EC 1.8.3.2), Polyphenoloxidase (EC 1.14.18.1), insbesondere Catecholoxidase, Tyrosinoxidase und Phenoloxidase, Lysyloxidase (EC 1.4.3.13) und Peroxidase (EC 1.11.1.7).

2. Mineralwollprodukt nach Anspruch 1, wobei das Kollagen oder die Gelatine aus einer oder mehreren Quellen aus der Gruppe bestehend aus Säugetieren, Vogelarten, wie z.B. von Kuh, Schwein, Pferd, Geflügel und/oder von Schuppen, Haut von Fisch stammt.

3. Mineralwollprodukt nach irgendeinem der vorhergehenden Ansprüche, wobei das Bindemittel in seinem ungehärteten Zustand einen pH-Einstellmittel umfasst, insbesondere in Form eines pH-Puffers.

4. Mineralwollprodukt nach irgendeinem der vorhergehenden Ansprüche, wobei das Bindemittel in seinem ungehärteten Zustand einen pH-Wert von weniger als 8, wie z.B. weniger als 7, wie z.B. weniger als 6, aufweist.

5. Mineralwollprodukt nach irgendeinem der vorhergehenden Ansprüche, wobei die Dichte des Mineralwollprodukts im Bereich von 10-900 kg/m$^3$, wie z.B. 30-800 kg/m$^3$, wie z.B. 40-600 kg/m$^3$, wie z.B. 50-250 kg/m$^3$, wie z.B. 60-200 kg/m$^3$, liegt.

6. Mineralwollprodukt nach irgendeinem der vorhergehenden Ansprüche, wobei der Glühverlust (LOI) im Bereich von 0,1 bis 25,0%, wie z.B. 0,3 bis 18,0%, wie z.B. 0,5 bis 12,0%, wie z.B. 0,7 bis 8,0%, bezogen auf das Gewicht, liegt.

7. Verfahren zur Herstellung eines Mineralwollprodukts, umfassend die Schritte des Inkontaktbringens von Mineralfasern mit einem Bindemittel nach irgendeinem der Ansprüche 1 bis 6 und des Härtens des Bindemittels, wobei das Härten bei Temperaturen von 5 bis 95 °C, wie z.B. 5 bis 80 °C, wie z.B. 8 bis 50 °C, wie z.B. 10 bis 40 °C, durchgeführt wird.

8. Verfahren zur Herstellung eines Mineralwollprodukts nach Anspruch 7, wobei das Verfahren die folgenden Schritte umfasst:

   - Bilden einer Schmelze von Rohstoffen,
   - Zerfasern der Schmelze mittels einer Faserbildungsvorrichtung zur Bildung von Mineralfasern,
   - Bereitstellen der Mineralfasern in Form einer gesammelten Bahn,
   - Mischen des Bindemittels mit den Mineralfasern vor, während oder nach der Bereitstellung der gesammelten Bahn, um eine Mischung aus Mineralfasern und Bindemittel zu bilden,
   - Härten der Mischung aus Mineralfasern und Bindemittel.

9. Verfahren zur Herstellung eines Mineralwollprodukts nach Anspruch 8, wobei das Mischen des Bindemittels mit den Mineralfasern nach dem Bereitstellen der gesammelten Bahn in den folgenden Schritten erfolgt:

   - Unterwerfen der gesammelten Bahn aus Mineralfasern einem Entwirrungsprozess,
   - Suspendieren der Mineralfasern in einem Primärluftstrom,
   - Mischen des Bindemittels mit den Mineralfasern vor, während oder nach dem Entwirrungsprozess, um eine Mischung aus Mineralfasern und Bindemittel zu bilden.

10. Verfahren zur Herstellung eines Mineralwollprodukts nach Anspruch 7-9, wobei der Härtungsprozess einen Trocknungsprozess umfasst, insbesondere durch Blasen von Luft oder Gas über das Mineralwollprodukt und/oder durch Erhöhen der Temperatur.

11. Verfahren zur Herstellung eines Mineralwollprodukts nach Anspruch 7-10, wobei das Härten in einer an Sauerstoff verarmten Umgebung durchgeführt wird.

**Revendications**

1. Produit en laine minérale comprenant des fibres minérales liées par un liant durci, où le liant dans son état non durci comprend :

   - au moins une protéine,
   - au moins une enzyme,
   où l'au moins une protéine est choisie dans le groupe constitué par les protéines d'origine animale, y compris le collagène, la gélatine, la gélatine hydrolysée, et les protéines du lait (caséine, lactosérum), des œufs ; les protéines d'origine végétale, y compris les protéines de légumineuses, céréales, grains entiers, noix, graines et fruits, telles que les protéines de sarrasin, avoine, seigle, millet, maïs (blé d'Inde), riz, blé, boulgour, sorgho, amarante, quinoa, soja (protéines de soja), lentilles, haricots secs, haricots blancs, haricots mungo, pois chiches, doliques, haricots de Lima, pois cajans, lupins, haricots ailés, amandes, noix du Brésil, noix de cajou, noix de pécan, noix, graines de coton, graines de potiron, graines de chanvre, graines de sésame, et graines de tournesol ; les protéines polyphénoliques telles que les protéines de pied de moule,
   où l'au moins une enzyme est choisie dans le groupe constitué par la transglutaminase (EC 2.3.2.13), la protéine disulfure isomérase (EC 5.3.4.1), la thiol oxydase (EC 1.8.3.2), la polyphénol oxydase (EC 1.14.18.1), en particulier la catéchol oxydase, la tyrosine oxydase, et la phénol oxydase, la lysyle oxydase (EC 1.4.3.13), et la peroxydase (EC 1.11.1.7).

**2.** Produit en laine minérale selon la revendication 1, où le collagène ou la gélatine est issu d'une ou plusieurs sources du groupe constitué par les mammifères, les espèces aviaires, telles que de vache, cochon, cheval, volaille, et/ou d'écaillés, de peau de poisson.

**3.** Produit en laine minérale selon l'une quelconque des revendications précédentes, où le liant dans son état non durci comprend un agent d'ajustement du pH, en particulier sous la forme d'un tampon de pH.

**4.** Produit en laine minérale selon l'une quelconque des revendications précédentes, où le liant dans son état non durci a une valeur de pH inférieure à 8, telle qu'inférieure à 7, telle qu'inférieure à 6.

**5.** Produit en laine minérale selon l'une quelconque des revendications précédentes, où la masse volumique du produit en laine minérale est située dans la plage de 10 à 900 kg/m$^3$, telle que 30 à 800 kg/m$^3$, telle que 40 à 600 kg/m$^3$, telle que 50 à 250 kg/m$^3$, telle que 60 à 200 kg/m$^3$.

**6.** Produit en laine minérale selon l'une quelconque des revendications précédentes, où la perte au feu (LOI) est située dans la plage de 0,1 à 25,0 %, telle que 0,3 à 18,0 %, telle que 0,5 à 12,0 %, telle que 0,7 à 8,0 % en poids.

**7.** Procédé de production d'un produit en laine minérale, qui comprend les étapes de mise en contact de fibres minérales avec un liant selon l'une quelconque des revendications 1 à 6, et de durcissement du liant, où le durcissement est effectué à des températures de 5 à 95°C, telles que 5 à 80°C, telles que 8 à 50°C, telles que 10 à 40°C.

**8.** Procédé de production d'un produit en laine minérale selon la revendication 7, où le procédé comprend les étapes de :

    - réalisation d'une masse fondue de matières premières,
    - mise de la masse fondue sous la forme de fibres au moyen d'un dispositif de formation de fibres pour former des fibres minérales,
    - mise des fibres minérales sous la forme d'une toile collectée,
    - mélange du liant avec les fibres minérales avant, pendant ou après la mise sous forme d'une toile collectée pour former un mélange de fibres minérales et de liant,
    - durcissement du mélange de fibres minérales et de liant.

**9.** Procédé de production d'un produit en laine minérale selon la revendication 8, où le mélange du liant avec les fibres minérales est effectué après la mise sous forme d'une toile collectée, dans les étapes suivantes :

    - soumission de la toile collectée de fibres minérales à un traitement de désenchevêtrement,
    - mise en suspension des fibres minérales dans un courant d'air primaire,
    - mélange du liant avec les fibres minérales avant, pendant ou après le traitement de désenchevêtrement pour former un mélange de fibres minérales et de liant.

**10.** Procédé de production d'un produit en laine minérale selon les revendications 7 à 9, où le traitement de durcissement comprend un traitement de séchage, en particulier par soufflage d'air ou de gaz sur le produit en laine minérale et/ou par augmentation de la température.

**11.** Procédé de production d'un produit en laine minérale selon les revendications 7 à 10, où le durcissement est effectué dans un environnement appauvri en oxygène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 583086 A
- EP 990727 A
- EP 1741726 A
- US 5318990 A
- US 20070173588 A
- WO 9936368 A
- WO 0105725 A
- WO 0196460 A
- WO 0206178 A
- WO 2004007615 A
- WO 2006061249 A
- WO 2008023032 A
- EP 2424886 B1
- WO 2010125163 A1
- WO 2016005481 A1
- GB 926749 A
- US 3824086 A
- WO 8303092 A
- EP 10190521 A

### Non-patent literature cited in the description

- **J. J. WILKER.** *Nature Chem. Biol.,* 2011, vol. 7, 579-580